# FASCICULE DE BREVET EUROPEEN

(11) **EP 1 613 738 B1**
(45) Date de publication et mention de la délivrance du brevet: **11.04.2012**
(21) Numéro de dépôt: 04727007.9
(22) Date de dépôt: 13.04.2004
(51) Int. Cl.: C12N 1/18, A21D 8/04

(54) **CONDITIONNEMENT DE LEVURE**
HEFE KONDITIONIERUNG
YEAST PACKAGING

(30) Priorité: 10.04.2003 EP 03008349; 23.12.2003 EP 03029589
(43) Date de publication de la demande: 11.01.2006
(73) Titulaire: Lesaffre et Compagnie, 75001 Paris (FR); Algist Bruggeman N.V., 9000 Gent (BE)
(72) Inventeur: SMET, Peter, René, Anna, B-9100 Sint-Niklaas (BE); BLOMME, Karel, Alfons, Frans, B-9940 Evergem (BE); VAN EETVELDE, Gabriel, Petrus, B-9940 Evergem (BE)
(74) Mandataire: Warcoin, Jacques
(86) Numéro de dépôt international: PCT/EP2004/003890
(87) Numéro de publication internationale: WO 2004/090116

(56) Documents cités:
- EP-A- 1 213 347
- WO-A-92/04037
- US-B1- 6 306 391
- SCEVOLA D ET AL: "Acid tolerance and fecal recovery following oral administration of Saccharomyces cerevisiae." JOURNAL OF CHEMOTHERAPY, vol. 15, no. 2, avril 2003 (2003-04), pages 143-147, XP009035604 ISSN: 1120-009X
- "LievitoVit Program" SITE INTERNET BENESSERE.COM, [Online] XP002293855 Extrait de l'Internet: URL:http://www.benessere.com/lievitovit/li e_prog_scheda.htm> [extrait le 2004-08-24]

## Description

La présente invention concerne l'utilisation d'une forme de conditionnement de levure sèche active, et notamment de levure sèche active de boulangerie, dans la préparation d'une pâte pour produit cuit.

La levure de boulangerie est commercialisée sous forme liquide, sous forme semi-humide et sous forme sèche.

La levure liquide ou crème de levure peut se conserver jusqu'à 28 jours, si elle est maintenue à une température de 0 à 7°C.

La levure émiettée et la levure pressée en blocs -levures semi-humides10 peuvent se conserver jusqu'à 31 jours, si elles sont maintenues à une température de 0 à 10°C.

La levure sèche active, instantanée ou non instantanée, en poudre, en vermicelles, sphérules ou granules fins, peut se conserver pendant au moins 2 ans à température ambiante (20 à 25°C) si elle est conservée à l'abri de l'humidité, et notamment l'humidité de l'atmosphère ambiante, et de préférence de l'oxygène et de la lumière.

Le fait que la levure sèche active peut être conservée à température ambiante durant une période beaucoup plus longue que la levure liquide ou crème de levure et la levure pressée ou émiettée est un des avantages principaux de la levure sèche active. Sa longue durée de conservation fait notamment que la levure sèche active est la forme de levure de boulangerie préférée par les utilisateurs qui, comme les ménagères, n'ont qu'occasionnellement besoin d'une faible quantité de levure de boulangerie.

La conservation de la levure sèche active pendant plusieurs mois, surtout sous la forme de levure sèche instantanée, nécessite, comme mentionné ci-dessus, qu'elle soit stockée à l'abri de l'humidité et de l'oxygène.

Ceci est actuellement réalisé par le conditionnement de la poudre, des vermicelles ou des granules fins de levure sèche active instantanée en des sacs, pochettes ou sachets étanches, soit sous vide, soit sous atmosphère sèche artificielle, comme par exemple sous atmosphère d'azote.

Afin de permettre une bonne conservation de la levure sèche active, le matériau desdits sacs, pochettes ou sachets doit répondre aux exigences minimales suivantes :
■ le matériau doit d'abord, bien évidemment être hautement étanche à l'humidité et à l'oxygène,
■ le matériau doit être approprié pour les produits alimentaires,
■ le matériau doit être souple et pliable et résister à la perforation et au déchirement, et
■ le matériau doit être soudable en donnant une soudure solide et étanche.

De préférence, le matériau doit être opaque de manière à protéger la levure sèche active de l'effet nuisible de la lumière.

Actuellement, la levure sèche active instantanée est conditionnée dans des sacs, pochettes ou sachets en matériau opaque multicouche, contenant au moins une couche métallique ou métallisée, de qualité et prix élevés, tels que décrits aux pages 306-307 du livre de référence « Yeast Technology » de Reed et Nagodawithana, deuxième édition 1991 (ISBN 0-442-31892-8).

Les résultats de conservation obtenus avec de tels sacs, pochettes ou sachets se sont en général avérés positifs.

Un problème se pose toutefois pour ce qui concerne le conditionnement de faibles quantités de levure sèche active, comme par exemple des doses de levure sèche active de 25 grammes ou moins, et en particulier en ce qui concerne le conditionnement d'une dose de levure sèche active correspondant à la quantité nécessaire pour la préparation d'un seul pain ou produit analogue. En effet, dans ce cas, le prix de l'emballage équivaut ou dépasse le prix de fabrication de la faible quantité ou dose de levure sèche active, voire en est un multiple.

Des méthodes de conditionnement de levures sous forme de comprimés sont connues dans l'art antérieur (EP 1 213 347 ; WO 92/04037 ; US 6,306,391 ; Scevola et al., J Chemother, 15 : 143-147, 2003).La présente invention concerne l'utilisation dans la préparation d'une pâte pour produit cuit d'un agent de fermentation contenant de la levure sèche active, ledit agent de fermentation étant sous la forme d'un comprimé.

Parmi les levures sèches actives, on distingue la levure sèche active de panification instantanée et la levure sèche active de panification non instantanée. La levure sèche active instantanée (en anglais : Instant Active Dry Yeast ou IADY ou IDY) se distingue notamment de la levure sèche active non instantanée (en anglais Active Dry Yeast ou ADY), par le fait que, pour avoir une activité élevée, la levure sèche active non instantanée doit être réhydratée avant son introduction dans la pâte boulangère, alors que la levure sèche active instantanée est un agent de fermentation qui ne nécessite pas une telle réhydratation (voir, par exemple, pages 297-304 du livre de référence « Yeast Technology » de Reed et Nagodawithana, deuxième édition 1991 (ISBN 0-442-31892-8)).

Le terme « comprimé » tel qu'utilisé dans le présent contexte, se réfère à une masse solide compacte de forme quelconque. Le comprimé suivant l'invention peut être obtenu par coulage et/ou compression dans un moule, par granulation, par agglomération ou par d'autres procédés connus, notamment en pharmacie, pour la préparation de tablettes, pilules, etc.

L'agent de fermentation suivant l'invention a une teneur en matières sèches d'au moins 88%, de préférence de 88 à 98% en masse, encore de préférence d'au moins 90% en masse, et plus de préférence de 90 à 98% en masse, et plus de préférence encore de 93 à 96% en masse.

La masse du comprimé peut varier de 1,0 g à 250,0 g.

Suivant une forme de réalisation, l'agent de fermentation présente une teneur en matières sèches de 90 à 96 % en masse. Dans ce cas, l'agent de fermentation contient typiquement de la levure sèche active non instantanée.

Suivant une deuxième forme de réalisation, l'agent de fermentation présente une teneur en matières sèches de 94 à 97 % en masse. Dans ce cas, l'agent de fermentation contient typiquement de la levure sèche active instantanée.

De manière avantageuse, l'équivalent de 160 mg de matières sèches de l'agent de fermentation, c'est-à-dire du comprimé, donne dans le test A1 décrit ci-après une production de CO₂ en 2 heures de 20 à 150 ml, de préférence de 30 à 130 ml, et encore de préférence de 40 à 120 ml, et suivant une forme particulièrement préférée de 90 ml à 170 ml.

De manière utile, l'équivalent de 160 mg de matières sèches de l'agent de fermentation, c'est-à-dire du comprimé, donne dans le test A2 décrit ci-après une production de CO₂ en 2 heures de 15 à 120 ml, de préférence de 25 à 110 ml, et encore de préférence de 35 à 100 ml, et suivant une forme particulièrement préférée de 90 ml à 170 ml.En particulier, l'équivalent de 160 mg de matières sèches de l'agent de fermentation peut donner, dans les tests A1 et A2, une production de CO₂ en deux heures de 20 à 150 ml, de préférence de 30 à 130 ml, et encore de préférence de 40 à 120 ml et suivant une forme particulièrement préférée de 90 ml à 170 ml.

La quantité de CO₂ produite par l'agent de fermentation dans les tests A1 et A2 dépendra notamment de la souche de levure présente, du procédé d'obtention de la levure, de la nature et des quantités des autres ingrédients éventuellement présents dans ou sur le comprimé et du procédé de compactage utilisé.

De manière préférée, l'agent de fermentation présente une production de CO₂ élevée dans les tests A1 et/ou A2. Toutefois, des productions de CO₂ plus faibles sont parfaitement acceptables, comme notamment des productions de CO₂ en 2 heures dans le test A1 supérieures ou égales à 20 ml, mais inférieures ou égales à 85ml, inférieures ou égales à 75 ml ou encore inférieures ou égales à 60 ml, et/ou des productions de CO₂ en 2 heures dans le test A2 supérieures ou égales à 15 ml, mais inférieures ou égales à 75 ml, inférieures ou égales à 65 ml ou encore inférieures ou égales à 55 ml.

La masse des comprimés peut varier, notamment en fonction de l'utilisation ou du marché envisagé. La masse d'un comprimé est typiquement d'au moins 1,5 g ; d'au moins 2,0 g ; d'au moins 2,5 g ou encore d'au moins 3,0 g. L'agent de fermentation suivant l'invention peut ainsi être sous la forme d'un comprimé de 1,0 à 100,0 g ; de 1,5 à 50,0 g ; de 2,0 à 12,0 g ; ou encore de 2,0 à 5,0 g.

La teneur en levure sèche active de l'agent de fermentation suivant l'invention peut également varier. Ainsi, l'agent de fermentation consistera typiquement pour au moins 30% en masse de levure sèche active. De manière utile, l'agent de fermentation peut consister pour au moins 50% en masse, de préférence pour au moins 75% en masse, encore de préférence pour au moins 80% en masse et plus de préférence encore pour au moins 90% en masse de levure sèche active.

A part la levure sèche active, l'agent de fermentation suivant l'invention peut également comprendre d'autres ingrédients.

Ainsi, l'agent de fermentation peut avantageusement comprendre un ou plusieurs auxiliaires technologiques utiles pour le séchage des levures et/ou ayant un rôle d'améliorant de panification. L'agent de fermentation peut notamment comprendre un ou plusieurs améliorants de panification du groupe des agents antioxydants, par exemple l'acide ascorbique, des agents réducteurs, par exemple la L-cystéine, des enzymes, des émulsifiants, par exemple des esters de sorbitan, du gluten. En particulier, l'agent de fermentation peut comprendre un ou plusieurs améliorants de panification appartenant au groupe de l'acide ascorbique, des enzymes, de la cystéine, du gluten, des malts et des extraits de malt, des extraits de levure et des levures sèches inactives. Quand l'agent de fermentation comprend un ou plusieurs améliorants de panification enzymatiques, il comprend préférentiellement une ou plusieurs enzymes du groupe des α-amylases, protéases, xylanases ou pentosanases, amyloglucosidases, glucose oxydases, lipases et phospholipases.

L'agent de fermentation suivant l'invention comprend de manière utile un ou plusieurs émulsifiants, comme notamment les émulsifiants du groupe suivant : la lécithine, les mono- et diglycérides d'acides gras, les esters diacétyltartriques de mono- et diglycérides (en anglais : DATEM), les esters de polyglycérol, les esters de sorbitan, les stéaroyls-lactylates.

L'agent de fermentation suivant l'invention peut également comprendre un ou plusieurs excipients. Dans le présent contexte, le terme « excipient » se réfère à des substances qui, à cause de leur nature ou leur quantité, n'agissent ni en tant qu'agent de fermentation ni en tant qu'améliorant de panification, mais qui facilitent, par exemple, la préparation de l'agent de fermentation, sa mise en forme ou sa conservation. L'agent de fermentation peut ainsi comprendre un ou plusieurs excipients comme :
- les polysaccharides (comme, par exemple, le cellulose, et en particulier le cellulose microcristallin),
- les dérivés de cellulose (comme, par exemple, le méthylcellulose et le carboxyméthylcellulose),
- les gommes,
- les pectines,
- les monosaccharides (comme par exemple le fructose et le glucose),
- les disaccharides (comme, par exemple, les lactoses tels que l'α-lactose monohydraté et le β-lactose anhydrique, le saccharose (en anglais : sucrose)),
- les polyalcools (comme, par exemple, le xylitol, le manitol, le sorbitol, le glycérol),
- le (di)phosphate de calcium,
- le polyvinylpyrrolidone.

Les monosaccharides, comme notamment le glucose, et en particulier le fructose, sont des excipients préférés.

Suivant une forme de mise en oeuvre, l'agent de fermentation comprend, en tant qu'excipient, un ou plusieurs agents effervescents. La présence de tels agents effervescents facilite la désintégration de l'agent de fermentation dans la pâte ou, le • cas échéant, la mise en suspension de l'agent de fermentation avant son introduction dans la pâte. Des agents effervescents appropriés pour la présente invention sont, par exemple, le bicarbonate de sodium éventuellement en combinaison avec de l'acide citrique, de l'acide lactique et/ou de l'acide tartrique. D'autres agents effervescents alimentaires peuvent également être utilisés. De préférence, si les agents effervescents conduisent à un dégagement de gaz mesuré dans les tests A1 ou A2, ce dégagement de gaz dû aux agents effervescents sera inférieur à 50% du dégagement total de gaz mesuré dans les tests A1 ou A2.

La mise en suspension de l'agent de fermentation avant son introduction dans la pâte améliore et/ou facilite la bonne répartition dudit agent dans la pâte boulangère.

L'agent de fermentation suivant l'invention peut éventuellement comporter des compléments d'alimentation comme l'iode, une ou plusieurs vitamines, du fluor, des suppléments alimentaires minéraux, les tocophérols, des oligo-éléments, des oméga-3 acides gras, des fibres alimentaires, ou encore de la farine, du miel, de la mélasse de canne, etc ; des améliorants de goût tels que des substituts de sel, des édulcorants, des épices ; des arômes naturels ou synthétiques, tels que les arômes de vanille, de fleur d'oranger, de rhum, de fruits rouges, d'agrumes ; des agents de conservation, tels que des propionates ; et/ou des colorants pour la pâte et/ou pour le produit cuit, tels que le caramel ; sachant que certains ingrédients peuvent avoir des effets multiples.

De manière avantageuse, l'agent de fermentation est sous la forme d'un comprimé couvert d'une couche formant barrière à l'humidité et/ou à l'oxygène, c'est-à-dire formant barrière contre l'humidité et/ou l'oxygène atmosphérique.

Par exemple, cette barrière à l'humidité et/ou à l'oxygène peut être une couche d'émulsifiant(s) et/ou de matière(s) grasse(s) venant enrober l'intérieur du comprimé comprenant la levure sèche active. Cette couche externe peut comprendre d'autres auxiliaires technologiques ayant un rôle d'améliorant de panification.

Il est à noter que, lors de l'utilisation finale de l'agent de fermentation, une répartition aussi homogène que possible de la levure dans la pâte est recherchée, ce qui implique la désintégration du comprimé avant la préparation de la pâte ou au cours du pétrissage. La désintégration du comprimé avant la préparation de la pâte peut, par exemple, être une désintégration mécanique, par exemple entre les doigts ou les mains, ou une désintégration par réhydratation ou mise en suspension.

Dans le cas d'un comprimé couvert d'une couche formant barrière à l'humidité et/ou à l'oxygène, le matériau de la couche doit donc permettre une telle désintégration du comprimé. La couche formant barrière à l'humidité et/ou à l'oxygène peut ainsi être en un matériau permettant que la couche se désintègre lors du pétrissage de la pâte ou que la couche se désintègre ou se dissolve dans l'eau présente dans la pâte.

Il est également possible d'incorporer un tel matériau dans la masse de l'agent de fermentation, de manière à ce que la levure sèche active soit entourée du matériau formant barrière à l'humidité et/ou à l'oxygène à l'intérieur du comprimé.

De nombreux matériaux d'enrobage appropriés en tant que couche formant barrière d'humidité et/ou d'oxygène pour les comprimés suivant l'invention et procédés d'enrobage utiles sont connus notamment dans les domaines des sucreries et des médicaments. En pratique, on utilise des matériaux d'enrobage qui ne sont pas collants à des températures rencontrées lors du transport et stockage des agents de fermentation.

La barrière à l'humidité et/ou à l'oxygène a pour but de réduire le contact direct entre, d'une part, l'agent de fermentation, et en particulier la levure sèche active qu'il contient, et d'autre part, l'humidité et/ou l'oxygène dans l'atmosphère gazeuse entourant le comprimé. Il n'est donc pas indispensable que cette barrière soit absolument imperméable à l'humidité et/ou l'oxygène dans l'atmosphère. En pratique, une barrière à perméabilité limitée retardant considérablement la diffusion des molécules d'eau et/ou d'oxygène à travers la barrière peut être suffisante.

L'agent de fermentation suivant l'invention peut aussi être sous la forme d'un comprimé muni d'une ou plusieurs rainures facilitant sa division en plusieurs parties.

Il est possible d'incorporer l'agent suivant l'invention dans une farine de panification ou dans un mélange, comme par exemple un mélange pour pizza ou gaufre, contenant de la farine de panification et d'autres ingrédients, comme notamment des améliorants de panification.

Comme indiqué ci-dessus, la capacité de l'agent de fermentation suivant l'invention à produire du CO₂ peut être évaluée par les tests A1 et A2 ci-après. Le test A1 fait appel à un milieu à base de farine non sucré, le test A2 fait appel à un milieu à base de farine légèrement sucré.

### Test A1

Le volume de CO₂ dégagé par l'agent de fermentation suivant l'invention est mesuré avec le fermentomètre de Burrows et Harrisson par la méthode pour la mesure du volume de CO₂ dégagé par de la levure décrite aux pages 579 à 584 du livre de référence «Guide pratique d'analyses dans les industries de céréales» coordonné par B.Godon et W.Loisel - Ed Lavoisier - Tec & Doc - 1997 - ISBN. 2-7430-0123-2 - 2° édition - en utilisant le mode opératoire pour les levures séchées avec réhydratation de l'agent de fermentation à 38°C.

La farine est une farine de froment ayant un temps de chute Hagberg de 200 à 300 secondes (tel que défini aux pages 680 et 681 dudit livre de référence «Guide pratique d'analyses dans les industries de céréales»).

La quantité de 160 mg de matières sèches d'agent de fermentation correspond à la quantité de matières sèches d'agent de fermentation présente dans les 15 ml pipetés de la suspension aqueuse nécessaires au malaxage (1,066g x 15 ml / 100 ml) (voir page 583 dudit livre de référence «Guide pratique d'analyses dans les industries de céréales»).

### Test A2

Le volume de CO₂ dégagé par l'agent de fermentation est mesuré avec le fermentomètre de Burrows et Harrisson par la méthode décrite aux pages 579 à 584 du livre de référence « Guide pratique d'analyses dans les industries de céréales » mentionné ci-dessus, en utilisant le mode opératoire pour les levures séchées avec réhydratation de l'agent de fermentation à 38°C mentionné ci-dessus, avec la modification suivante : avant incubation des lots de 20 g de farine de froment, on transvase dans les tubes 2 g de saccharose.

La présente invention concerne aussi des procédés pour la production d'un agent de fermentation sous forme d'un comprimé, ledit agent de fermentation ayant une teneur en matières sèches d'au moins 88% en masse, de préférence d'au moins 90% en masse, et encore de préférence d'au moins 93% en masse, et encore plus de préférence d'au moins 94% en masse, et contenant de la levure sèche active, ainsi que les agents de fermentation ainsi obtenus.

L'invention concerne par exemple un procédé dans lequel le comprimé est formé par compactage. La pression de compactage peut varier. Elle peut, par exemple, être inférieure à 3x10⁸ Pa, de préférence de 0,5x10⁵ à 1x10⁸ Pa, et encore de préférence de 0,5x10⁵ à 5x10⁷ Pa.

De manière utile, le procédé comprend la préparation d'un mélange ayant une teneur en matières sèches d'au moins 88% en masse, de préférence d'au moins 90% en masse, encore de préférence d'au moins 93% en masse et plus de préférence encore d'au moins 94% en masse, et contenant de la levure sèche active, et la formation d'un comprimé du dit mélange, et en particulier la formation d'un comprimé par compactage dudit mélange à une pression de compactage telle que définie ci-dessus. Ladite levure sèche active peut être une levure sèche active instantanée et/ou non instantanée.

De manière avantageuse, dans le procédé ci-dessus, le mélange contenant de la levure sèche active est réalisé en pulvérisant un excipient, par exemple à base d'un monosaccharide, sur la levure sèche active, la levure sèche active étant par exemple maintenue en lit fluidisé lors de cette pulvérisation.

Des procédés, tels que décrits ci-dessus, se sont avérés particulièrement intéressants pour la production d'agents de fermentation suivant l'invention tels que définis ci-dessus.

La présente invention couvre donc l'utilisation d'un agent de fermentation suivant l'invention dans la préparation d'une pâte pour produit cuit.

L'invention concerne également des procédés pour la préparation d'une pâte pour produit cuit, procédé comprenant une étape de mélange de l'agent de fermentation selon l'invention avec d'autres ingrédients de la pâte, comme notamment la farine. L'agent de fermentation peut être mélangé en tant que tel avec d'autres ingrédients de la pâte, peut être mécaniquement (par exemple, manuellement) désintégré avant d'être mélangé avec d'autres ingrédients de la pâte ou encore peut être réhydraté avant d'être mélangé avec d'autres ingrédients.

L'invention concerne aussi des procédés pour la préparation de produits cuits, procédé comprenant une étape de préparation d'une pâte telle que décrite ci-dessus, une étape de fermentation de la pâte par l'agent de fermentation et une étape de cuisson de la pâte fermentée ainsi obtenue.

Les pâtes sont, par exemple, des pâtes pour les produits de boulangerie, en particulier pour des pains, pour les produits de pâtisserie, pour les produits de viennoiserie, pour les pizzas, pour les crêpes, pour les gaufres, etc.

La présente invention concerne également un conditionnement de levure *sèche* active qui comprend un ou plusieurs comprimés du présent agent de fermentation.

L'agent selon l'invention peut être conditionné dans des conditionnements divers, comme des conteneurs, de préférence en plastique, des enveloppes, etc.

Suivant une forme de réalisation du conditionnement suivant l'invention, le ou les comprimés sont individuellement enveloppés dans une feuille formant barrière à 1 "humidité et de préférence à l'oxygène. Les comprimés peuvent également être enveloppés à deux ou à trois, ou encore à plus de trois, dans une feuille formant barrière à l'humidité et de préférence à l'oxygène.

La feuille formant barrière à l'humidité et de préférence à l'oxygène est de préférence opaque.

Des feuilles formant barrière à l'humidité et de préférence à l'oxygène appropriées sont connues et sont par exemple décrites dans les documents WO-A-94/14151 et WO-A-95/25436.

Suivant une forme de mise en oeuvre pratique, le conditionnement comprend des comprimés qui sont des tablettes de forme et de taille en substance identiques, sachant qu'une tablette est un comprimé aplati. De préférence, dans le conditionnement une série de ces tablettes est alignée s'appuyant l'une contre l'autre, l'ensemble de cette série étant enveloppé par une enveloppe cylindrique.

Il est à noter que, dans le présent texte, le terme cylindrique est utilisé au sens large. Ce terme se réfère donc non seulement aux cylindres ayant une section transversale circulaire, mais également aux cylindres ayant une autre section transversale, comme par exemple une section transversale polygonale, telle qu'une section triangulaire, carrée, rectangulaire, hexagonale ou octogonale. L'enveloppe cylindrique peut ainsi être une enveloppe parallélépipédique. Les tablettes ont les formes appropriées correspondant aux dites enveloppes.

Cette enveloppe cylindrique est avantageusement une enveloppe formant barrière à l'humidité et de préférence à l'oxygène.

Cette enveloppe cylindrique peut être une feuille souple enveloppant l'ensemble de la série.

Ladite feuille souple peut, par exemple, être une feuille formant barrière à l'humidité et de préférence à l'oxygène telle que décrite dans les documents WO-A-94/14151 et WO-A-95/25436 cités ci-dessus, ou encore une feuille formant barrière à l'humidité et de préférence à l'oxygène telle que décrite dans le document EP-A-1247642.

Le conditionnement suivant l'invention peut également être un tube, typiquement en matière plastique ou en un autre matériau rigide, muni d'un bouchon, tube dans lequel se trouvent plusieurs comprimés. Le tube peut, de manière utile, également être muni d'un élément de dessiccation. De tels tubes munis d'un bouchon et de tels éléments de dessiccation sont notamment connus dans le domaine de la pharmacie. Le tube est de préférence opaque.

Le conditionnement suivant l'invention peut aussi comprendre un sachet contenant un ou plusieurs comprimés. Ledit sachet est de préférence formé en une matière formant barrière à l'humidité et de préférence à l'oxygène, et est aussi de préférence opaque.

Le conditionnement suivant l'invention peut aussi comprendre une feuille blister contenant plusieurs comprimés.

L'agent de fermentation suivant l'invention et son conditionnement sont notamment utiles comme agent de fermentation pour utilisation ménagère. Toutefois, l'agent de fermentation suivant l'invention et son conditionnement peuvent également être utilisés dans d'autres domaines d'application. Ainsi, l'agent de fermentation et/ou le conditionnement peuvent faire partie d'un ensemble contenant de la farine ou un mélange à base de farine, comme par exemple un mélange pour gaufres, et l'agent de fermentation.

### Exemple

Un mélange homogène est préparé avec les ingrédients suivants :
- 100 parties en poids de levure de boulangerie sèche active instantanée,
- 1 partie en poids de glycérol,
- 5 parties en poids de bicarbonate de sodium en tant qu'agent effervescent,
- 5 parties en poids d'acide citrique.

Des pastilles d'un diamètre de 2 cm et de poids de 2,22 g sont préparées par compactage sous une pression de 223kg/cm² (2x10⁷Pa). Chaque pastille contient une quantité du mélange qui correspond à 2 g de levure sèche instantanée.

Une pâte boulangère est préparée avec les ingrédients suivants :
- 1800 g de farine de froment,
- 1060 g d'eau,
- 36g de sel de table,
- 18 g d'améliorant de panification standard,
- 10 pastilles.

La pâte est pétrie dans un pétrisseur OASE^{®} Spiralkneter SPK8 de la société DIOSNA (Osnabrück, Allemagne) en première vitesse pendant 3 minutes, puis en deuxième vitesse jusqu'à ce que la température dans la pâte atteigne 26°C.

La pâte est divisée en trois pâtons de 900 g chacun.

Les pâtons sont mis en forme de boules qu'on laisse reposer pendant 30 minutes (première fermentation).

Les pâtons sont ensuite mis en forme allongée qu'on laisse reposer (seconde fermentation) jusqu'à obtention du volume voulu.

Apres la seconde fermentation, les pâtons sont mis au four et cuits à 200°C pendant 35 minutes.

Les pains cuits ont l'aspect (volume, couleur, structure) et les propriétés organoleptiques d'un pain analogue préparé avec de la levure sèche active instantanée traditionnelle. Aucun problème rhéologique ne s'est manifesté au cours de la préparation de la pâte.

## Revendications

1. Utilisation d'un agent de fermentation dans la préparation d'une pâte pour produit cuit, **caractérisé en ce que** ledit agent de fermentation :
• possède une teneur en matières sèches d'au moins 88% en masse ;
• contient de la levure sèche active ; et
• est sous forme d'un comprimé de 1,0 g à 250,0 g.

2. Utilisation suivant la revendication 1, **caractérisée en ce que** l'agent de fermentation possède une teneur en matières sèches de 90 à 96 % en masse et qu'il contient de la levure sèche active non instantanée.

3. Utilisation suivant la revendication 1, **caractérisée en ce que** l'agent de fermentation possède une teneur en matière sèches de 94 à 97 % en masse et qu'il contient de la levure sèche active instantanée.

4. Utilisation suivant l'une quelconque des revendications précédentes, **caractérisée en ce que** l'agent de fermentation est sous forme d'un comprimé de 1,0 à 100,0 g ; de préférence de 1,5 g à 50,0 g ; encore de préférence de 2,0 à 10,0 g ; et plus de préférence encore de 2,0 à 5,0 g.

5. Utilisation suivant l'une quelconque des revendications précédentes, **caractérisée en ce que** l'agent de fermentation consiste pour au moins 30% en masse, de préférence pour au moins 50% en masse, encore de préférence pour au moins 75% en masse et plus de préférence encore pour au moins 80% en masse, et encore plus de préférence pour au moins 90% en masse de levure sèche active.

6. Utilisation suivant l'une quelconque des revendications précédentes, **caractérisée en ce que** l'agent de fermentation contient également un ou plusieurs auxiliaires technologiques ayant un rôle lors du séchage de la levure et/ou ayant un rôle d'améliorant de panification.

7. Utilisation suivant la revendication 6, **caractérisée en ce que** l'agent de fermentation contient un ou plusieurs auxiliaires technologiques ayant un rôle d'améliorant de panification choisis dans le groupe des agents antioxydants, des agents réducteurs, des enzymes et des émulsifiants.

8. Utilisation suivant l'une quelconque des revendications précédentes, **caractérisée en ce que** l'agent de fermentation contient un ou plusieurs excipients.

9. Utilisation suivant la revendication 8, **caractérisée en ce que** l'agent de fermentation contient un ou plusieurs excipients choisis dans le groupe des polysaccharides, des dérivés de cellulose, des gommes, des pectines, des mono- et disaccharides, du phosphate de calcium et du diphosphate de calcium, du polyvinylpyrrolidone, des polyalcools.

10. Utilisation selon l'une quelconque des revendications 8 ou 9, **caractérisée en ce que** l'agent de fermentation contient un monosaccharide choisi dans le groupe du fructose et du glucose.

11. Utilisation suivant l'une quelconque des revendications précédentes, **caractérisé en ce que** le comprimé est couvert d'une couche formant barrière à l'humidité et/ou à l'oxygène.

12. Procédé pour la préparation d'une pâte pour produit cuit, ledit procédé comprenant une étape dans laquelle l'agent de fermentation selon l'une quelconque des revendications 1 à 11 est mélangé avec d'autres ingrédients de la pâte.

13. Procédé pour la préparation d'une pâte pour produit cuit, procédé comprenant une étape de préparation de pâte pour produit cuit selon le procédé suivant la revendication 12, une étape de fermentation de la pâte par l'agent de fermentation et une étape de cuisson de la pâte fermentée ainsi obtenue.

## Claims

1. Use of a fermentation agent in the preparation of a dough for a baked product, wherein said fermentation agent:
• has a dry matter content of at least 88% by weight;
• contains active dry yeast; and
• is in the form of the tablet of 1.0 g to 250.0 g in weight.

2. Use according to claim 1, wherein the fermentation agent has a dry matter content of 90% to 96% by weight and wherein it contains non-instant active dry yeast.

3. Use according to claim 1, wherein the fermentation agent has a dry matter content of 94% to 97% by weight and wherein it contains instant active dry yeast.

4. Use according to any one of the preceding claims, wherein the fermentation agent is in the form of a tablet of 1.0 g to 100.0 g; preferably of 1.5 g to 50.0 g; preferably still of 2.0 g to 10.0 g; and more preferably still of 2.0 g to 5.0 g.

5. Use according to any one of the preceding claims, wherein the fermentation agent consists of at least 30% by weight, preferably of at least 50% by weight, preferably still of at least 75% by weight, and more preferably still of at least 80% by weight, and even more preferably of at least 90% by weight of active dry yeast.

6. Use according to any one of the preceding claims, wherein the fermentation agent also contains one or more auxiliary technological means having a role during the drying of yeast and/or having a role as a bread improver.

7. Use according to claim 6, wherein the fermentation agent contains one or more auxiliary technological means having a role as a bread improver chosen from among the group of antioxidant agents, reducing agents, enzymes, and emulsifiers.

8. Use according to any one of the preceding claims, wherein the fermentation agent contains one or more excipients.

9. Use according to claim 8, wherein the fermentation agent contains one or more excipients chosen from among the group of polysaccharides, cellulose derivatives, gums, pectins, monosaccharides and disaccharides, calcium phosphate and calcium diphosphate, polyvinylpyrrolidone, and polyalcohols.

10. Use according to any one of claim 8 or claim 9, wherein the fermentation agent contains a monosaccharide chosen from among the group of fructose and glucose.

11. Use according to any one of the preceding claims, wherein the tablet is covered with a layer that forms a barrier against moisture and/or oxygen.

12. A process for the preparation of a dough for a baked product, the said process including a step in which the fermentation agent according to any one of the claims 1 to 11 is mixed with other dough ingredients.

13. A process for the preparation of a dough for a baked product, the said process including a step of preparing the dough for a baked product according to the process according to claim 12, a step of fermenting the dough by the fermentation agent and a step of baking the fermented dough thus obtained.

## Patentansprüche

1. Verwendung eines Fermentationsmittels bei der Herstellung eines Teigs für ein gebackte Produkt, **dadurch gekennzeichnet, dass** das Fermentationsmittel:
• einen Trockensubstanzgehalt von mindestens 88 Massenprozent aufweist,
• aktive Trockenhefe enthält und
• in Form einer Tablette von 1,0 g bis 250,0 g vorliegt.

2. Verwendung gemäß Anspruch 1, **dadurch gekennzeichnet, dass** das Fermentationsmittel einen Trockensubstanzgehalt von 90 bis 96 Massenprozent aufweist und aktive Nicht-Instant-Trockenhefe enthält.

3. Verwendung gemäß Anspruch 1, **dadurch gekennzeichnet, dass** das Fermentationsmittel einen Trockensubstanzgehalt von 94 bis 97 Massenprozent aufweist und aktive Instant-Trockenhefe enthält.

4. Verwendung gemäß irgendeinem der vorhergehenden Ansprüche, **dadurch gekennzeichnet, dass** das Fermentationsmittel in Form einer Tablette von 1,0 bis 100,0 g, bevorzugt von 1,5 g bis 50,0 g, noch bevorzugter von 2,0 bis 10 g und am Bevorzugtesten von 2,0 bis 5,0 g, vorliegt.

5. Verwendung gemäß irgendeinem der vorhergehenden Ansprüche, **dadurch gekennzeichnet, dass** das Fermentationsmittel zu wenigstens 30 Massenprozent, bevorzugt zu wenigstens 50 Massenprozent, noch bevorzugter zu wenigstens 75 Massenprozent, noch bevorzugter zu wenigstens 80 Massenprozent und am Bevorzugtesten zu wenigstens 90 Massenprozent, aus aktiver Trockenhefe besteht.

6. Verwendung gemäß irgendeinem der vorhergehenden Ansprüche, **dadurch gekennzeichnet, dass** das Fermentationsmittel außerdem einen oder mehrere technologische Hilfsstoffe enthält, die eine Rolle bei der Trocknung der Hefe und/oder eine Rolle bei der Verbesserung der Brotbereitung spielen.

7. Verwendung gemäß Anspruch 6, **dadurch gekennzeichnet, dass** das Fermentationsmittel einen oder mehrere technologische Hilfsstoffe enthält, die eine Rolle bei der Verbesserung der Brotbereitung spielen und ausgewählt sind aus der Gruppe von Antioxidantien, Reduktionsmitteln, Enzymen und Emulgatoren.

8. Verwendung gemäß irgendeinem der vorhergehenden Ansprüche, **dadurch gekennzeichnet, dass** das Fermentationsmittel einen oder mehrere Hilfstoffe enthält.

9. Verwendung gemäß Anspruch 8, **dadurch gekennzeichnet, dass** das Fermentationsmittel einen oder mehrere Zusatzstoffe enthält, die ausgewählt sind aus der Gruppe von Polysacchariden, Zellulosederivaten, Gummis, Pektinen, Mono- und Disacchariden, Calciumphosphat und Calciumdiphosphat, Polyvinylpyrrolidon und Polyalkoholen.

10. Verwendung gemäß irgendeinem der Ansprüche 8 oder 9, **dadurch gekennzeichnet, dass** das Fermentationsmittel ein Monosaccharid enthält, das ausgewählt ist aus der Gruppe von Fruktose und Glukose.

11. Verwendung gemäß irgendeinem der vorhergehenden Ansprüche, **dadurch gekennzeichnet, dass** die Tablette mit einer Schicht überzogen ist, welche eine Barriere gegen Feuchtigkeit und/oder Sauerstoff bildet.

12. Verfahren zur Herstellung eines Teigs ein gebackfe Produkt, wobei das Verfahren einen Schritt umfasst, in dem das Fermentationsmittel gemäß irgendeinem der Ansprüche 1 bis 11 mit anderen Zutatenen des Teigs vermengt ist.

13. Verfahren zur Herstellung eines Teigs für ein gebackfe Produkt, wobei das Verfahren einen Schritt der Herstellung des Teigs für ein gebackfe Produkt gemäß dem Verfahren von Anspruch 12, einen Schritt der Fermentation des Teigs durch das Fermentationsmittel und einen Schritt des Backens des so erhaltenen fermentierten Teigs umfasst.
